Europäisches Patentamt

European Patent Office

Office européen des brevets

(11) Publication number: **0 204 954 B1**

(12) **EUROPEAN PATENT SPECIFICATION**

(45) Date of publication of patent specification: **22.07.92**   (51) Int. Cl.⁵: **A61K 31/135**, A61K 31/195, A61K 31/48

(21) Application number: **86106140.6**

(22) Date of filing: **05.05.86**

(54) **Use of dopamine agonists for the manufacture of a medicament for the treatment of psychostimulant addiction.**

(30) Priority: **06.05.85 US 731102**
**25.10.85 US 791188**

(43) Date of publication of application:
**17.12.86 Bulletin 86/51**

(45) Publication of the grant of the patent:
**22.07.92 Bulletin 92/30**

(84) Designated Contracting States:
**AT BE CH DE FR GB IT LI LU NL SE**

(56) References cited:
**US-A- 3 995 058**

**JOURNAL OF NEUROSCIENCE RESEARCH, vol. 3, 1977, pages 295-300, Alan R. Liss, Inc. New-York, US; M. LYNCH et al.: "The effect of chronic administration of D-amphetamine on regional changes in catecholamines in the rat brain"**

**PHARMACOLOGIST, vol. 17, no. 288, 1975, US; G.M. EVERETT: "A unique dopaminemimetic: pemoline"**

(73) Proprietor: **SANDOZ AG**
**Lichtstrasse 35**
**CH-4002 Basel(CH)**

(84) Designated Contracting States:
**BE CH FR GB IT LI LU NL SE**

(73) Proprietor: **SANDOZ-PATENT-GMBH**
**Humboldtstrasse 3**
**W-7850 Lörrach(DE)**

(84) Designated Contracting States:
**DE**

(73) Proprietor: **SANDOZ-ERFINDUNGEN Verwaltungsgesellschaft m.b.H.**
**Brunner Strasse 59**
**A-1235 Wien(AT)**

(84) Designated Contracting States:
**AT**

(72) Inventor: **Dackis, Charles Andrew**
**96 Joan Drive**
**Watchung N.J., 07060(US)**
Inventor: **Gold, Mark Steven**
**145 Forest Drive**
**Short Hills N.J.(US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

PSYCHOPHARMACOLOGY, vol. 58, 1978, pages 131-136, Springer Verlag, Heidelberg, DE; P. JENNER et al.: "The effect of chronic administration and withdrawal of amphetamine on cerebral dopamine receptor sensivity"

JOURNAL OF CLINICAL PSYCHIATRY, vol. 44, 1983, pages 206-208, US; J. D. GRIFFITH et al.: "Bupropion: clinical assay for amphetamine-like abuse potential"

PSYCHOPHARMACOLOGY, vol. 80, 1983, pages 199-205, Springer-Verlag, Heidelberg, DE; L.MILLER et al.: "A comparison of bupropion, dextroamphetamine, and placebo in mixed-substance abusers"

J. SUBSTANCE ABUSE TREATMENT, vol. 2, 1985, pages 139-146, Pergamon Press Ltd, US; C. A. DACKIS et al.: "Pharmacological approaches to cocaine addi ction"

J.E.F. Reynolds: MARTINDALE, The Extra Pharmacopoeia, 28th Edition, 1982, The Pharmaceutical Press, London, GB;

INTERNATIONAL JOURNAL OF PSYCHIATRY IN MEDICINE, vol. 15, no.2, 1985-1986, pages 125-135, Baywood Publishing Co. Ltd., US; C.A. DACKIS et al.: "Bromocriptine treatment for cocaine abuse: the dopamine depletion hypothesis"

NEUROSCIENCE AND BIOBEHAVIORAL REVIEWS, vol. 9, 1985, pages 469-477, Amkho International Inc., US; C.A. DACKIS et al.: "New concepts in cocaine addiction: the dopamine depletion hypothesis"

## Description

The present invention relates to a new use of dopamine agonists.

More particularly the present invention relates to the use of dopamine agonists for treating psychostimulant addiction, in particular for treating withdrawal symptoms in patients under-going treatment for psychostimulant abuse and for preventing craving for psychostimulants after withdrawal.

Psychostimulant abuse has grown rapidly over the years, evolving into a major medical and social problem. The most commonly abused psychostimulants are cocaine, amphetamine, methamphetamine, dextroamphetamine, pemoline and their pharmaceutically acceptable acid addition salts such as the phosphate, sulphate, 4-chloro-phenoxyacetate and the like.

In the case of cocaine for example it is estimated that initial and compulsive use soars at a rapid rate. For example in the USA this increased use has led to a threefold rise in emergency room visits, a fourfold increase in deaths and a sixfold increase in hospitalization between 1976 and 1981.

Medical complications of psychostimulant abuse comprise only one element of this hazardous practice. Financial, legal, social and vocational deterioration often occurs as compulsive cocaine use becomes the user's highest priority.

Abrupt cessation of chronic high-dose of psychostimulants can result in a variety of undesirable physical symptoms. Cocaine withdrawal, for instance, although this stimulant does not produce a stereotyped abstinence syndrome as with opiates or barbiturates, induces symptoms including: drug craving, depression, irritability, anergia, amotivation, appetite changes, nausea, shaking, psychomotoric retardation and irregular sleep patterns hypersomnia that may persist for a week or longer following cessation of use. Relief of these symptoms would allow patients to stop using psychostimulants more easily and would also be useful in the post-drug recovery period.

It has been widely reported that cocaine is a potent inhibitor of dopamine reuptake and appears to cause acute increases in dopamine transmission. Dopaminergic neurons also appear to mediate the euphoric response to cocaine and are critical in the development of addiction. Certain effects of cocaine depend on the integrity of the dopaminergic systems. Animals in which a lesion in the nucleus accumbens is produced by the dopamine toxin 6-hydroxy-dopamine, stop self-administering cocaine. However, even though cocaine at first increases dopamine transmission and concentration, a functional reduction in dopaminergic activity appears to follow administration of the drug.

It has also been reported that dopamine receptor agonists, such as apomorphine and piribedil, have amphetamine-like rewarding action in animals. Conversely, selective dopamine receptor antagonists reduce or eliminate both electrical and central stimulant self-administration by animals. The antagonist pimozide initially caused animals to increase lever pressing for amphetamine at low doses; but at increased doses, it obliterated the self-administration of amphetamine. These results illustrate the part played by dopamine in the mechanism of reward.

It is now proposed that psychostimulants in general at first increase dopamine transmission and concentration in humans, following which there is a functional reduction in dopaminergic activity. This reduction results in a craving for more psychostimulant as a means of increasing dopamine and relieving the desire for the drug.

In the present invention, it has been found that psychostimulant abuse can be treated by administering to a human abuser a dopamine agonist in an amount which is effective in inhibiting or eliminating the need for the drug. More particularly, it has been found that the withdrawal symptoms resulting from the abrupt cessation of chronic high dose use of psychostimulants can be significantly reduced or eliminated by the administration of an effective amount of a dopamine agonist. The dopamine agonist can be any dopamine stimulating agent, for example, levodopa, bromocriptine, bupropion, pergolide, lisuride and lergotrile, to name a few. The preferred dopamine agonist in the invention is bromocriptine. The amount of agonist administered will vary depending on the active agent used and the patient undergoing treatment. Essentially it will be the same as the known therapeutic dosage. It has however been surprisingly found that particularly good results are obtained in the majority of cases with daily dosages significantly lower than those administered for their known indications. The therapeutically effective amount of levodopa is from 0.5 to 1.0 gram per day. Bromocriptine is generally administered in dosages ranging from 0.1 to 10 milligrams per day. Pergolide and lisuride are effective at daily doses of about 0.35 to 1.0 milligram. Generally, the daily dosage for the ergolene and ergoline type dopamine agonist will be from about 0.05 to about 20 milligrams per day. The daily dosage for non-ergoline type agonists will normally be from 5 to 1000 milligrams per day. The agonist can be administered on a set schedule, for example, every 4 to 6 hours, or it can be administered in accordance with the patient's requirement. The preferred agonist, bromocriptine, is administered in unit doses of about 0.5 to 2.5 milligrams 2 to 4 times daily, preferably about 0.5 to 1.0 milligram 2 to 3 times daily. Normally, a unit dose of about 0.5 to 1 milligram of bromocriptine as required is sufficient to reduce or eliminate the desire for the psychostimulant being abused. It will be appreciated that such doses are significantly lower than those administered for the known indications of this drug, which range from about 2.5 to about 50 mg daily.

When appropriate, the dopamine agonist used in the invention may be employed in free base form or in pharmaceutically acceptable salt form. The various ergolene derivatives referred to above may, for example, be used in the form of the mesylate or hydrochloride salt. Generally the activities of such salt forms will be of the same order as that of the respective free form, and references to compounds in the free form throughout the specification and claims are to be understood as including known salt forms.

The active agent of the invention may be administered orally or parenterally as such or admixed with conventional pharmaceutical carriers. They may be administered orally in such forms as tablets, dispersible powders, granules, capsules, syrups and elixirs, and parenterally as solutions, e.g., a sterile injectable aqueous solution. Tablets may contain the active ingredients in admixture with conventional pharmaceutically acceptable excipients, e.g., inert diluents and granulating, disintegrating and lubricating agents. The tablets may be uncoated or coated by known techniques to delay disintegration and absorption in the gastrointestinal tract and thereby provide a sustained action over a longer period. Similarly, suspensions, syrups and elixirs may contain the active ingredients in admixture with any of the conventional excipients utilized in the preparation of such compositions. Capsules may contain the active ingredients alone or admixed with an inert solid diluent. The injectable compositions are formulated as known in the art. These pharmaceutical preparations nay contain up to about 90 % of the active ingredients in combination with the carrier or adjuvant.

In one patient study, nine patients, four male and five female, ranging in age from 18 to 34, were treated for cocaine withdrawal symptoms with commercially available bromocriptine mesylate. The average length of addiction was four years. Approximately one hour after the last use of the drug, abstinence symptoms began and reached their peak in three days. After admission to the study, the nine patients were given 0.625 milligrams p.o. of bromocriptine mesylate. The withdrawal symptoms subsided in all patients for periods ranging from 4.5 to 6 hours. Repeated dosing for 1 to 2 weeks permitted the patients to remain free of the most serious symptoms and eventually to enter the group therapy stage of the recovery program.

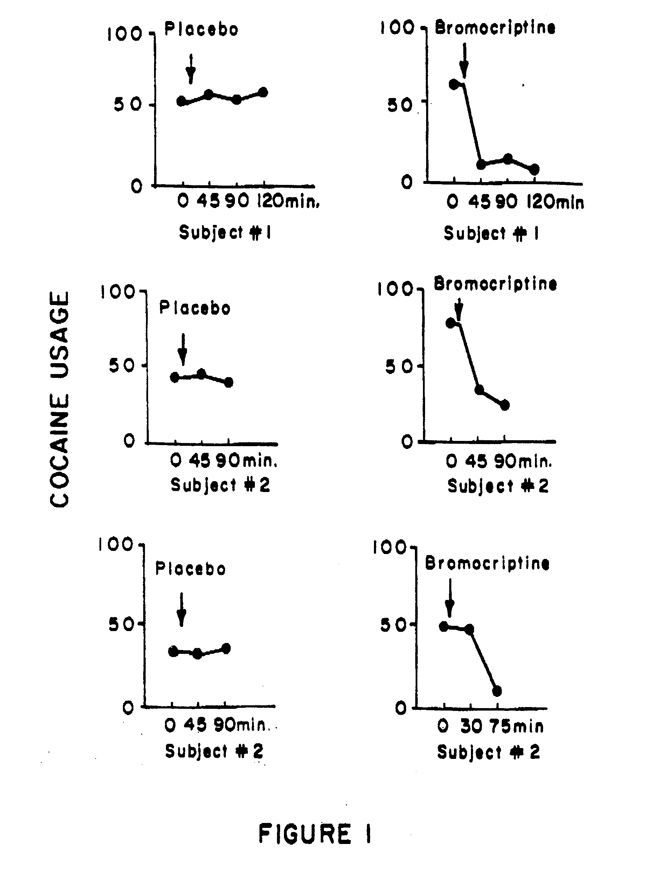

In a second study, two female in-patients, ages 18 and 20, gave informed consent to receive either bromocriptine or placebo. Both patients were contemplating leaving the hospital to use cocaine, and each complained of depressed mood, anergia, suicidal ideation, and poor concentration. The diagnosis of cocaine abuse was established after a complete evaluation and comprehensive testing to rule out other drug abuse and any medical illness. The patients were asked to rate the degree of craving for cocaine by marking a 130 millimeter line anywhere between "not at all" and "extremely". Scores were assigned according to the point marked, ranging between 0 and 100. Craving was again self-rated at varying time points after 0.625 milligrams p.o. of bromocriptine mesylate or placebo were administered blindly. The results of the cocaine urge self rating before and after placebo or bromocriptine administration is shown in Figure 1. As can be seen from the figure, both patients reported marked and consistent relief from cocaine craving after taking bromocriptine. In patient No. 1, the cocaine urge self rating dropped frown about 60 to about 5 in two hours upon administration of the bromocriptine. In patient Nr. 2, the cocaine urge self rating dropped from about 75 to about 20 in 90 minutes upon administration of bromocriptine in one rating session, and from about 50 to about 5 in 75 minutes in another session. In addition, both patients were able to distinguish bromocriptine from placebo in all six trials. Afterwards, Subject 1 was given the dopamine antagonist, thioridazine, for the treatment of visual hallucinations, and reported an acute increase in cocaine craving. When the effect of the dopamine antagonist on cocaine urges was assessed, craving in two trials increased from baseline scores of 7 and 15 to scores of 30 and 70 respectively after 30 minutes. The results of the study show that cocaine withdrawal symptoms are significantly reduced by the administration of a dopamine agonist, while cocaine urges are exacerbated by dopamine antagonists.

In a further study, a patient was treated for amphetamine abuse with commercially available bromocriptine mesylate. Shortly after the last use of amphetamine, abstinence symptoms began. The patient was given 0.625 milligrams p.o. of bromocriptine mesylate, and withdrawal symptoms subsided. Repeated dosing as needed permitted the patient to remain free of the most serious symptoms.

The present invention thus provides the use of a dopamine agonist for the manufacture of a pharmaceutical composition for the treatment of psychostimulant addiction.

Tablets having a diameter of 6 mm and the following composition may be prepared according to conventional methods and are suitable for use in the treatment of psychostimulant addiction.

0.574 mg of bromocriptine mesilate correspond to 0.5 mg of the free base form.

4

| Bromocriptine mesylate | 0.574 mg |
|---|---|
| Disodium salt of ethylene diamine tetraacetic acid 2H$_2$O | 0.325 mg |
| Silicium dioxide (Aerosil 200) | 0.225 mg |
| Lactose | 71.076 mg |
| Magnesium stearate | 0.450 mg |
| Corn starch | 11.700 mg |
| Maleic acid | 0.650 mg |
| | 85.000 mg |

## Claims

1. The use of a dopamine agonist for the manufacture of a pharmaceutical composition for the treatment of psychostimulant addiction.

2. The use according to claim 1, in which the psychostimulant is selected from amphetamine, dextroamphetamine, methamphetamine and pemoline or a pharmaceutically acceptable acid addition salt thereof.

3. The use according to claim 1, in which the psychostimulant is cocaine.

4. The use according to any one of claims 1 to 3, in which the dopamine agonist is selected from levodopa, bupropion, pergolide, lisuride or lergotrile.

5. The use according to any one of claims 1 to 3, in which the dopamine agonist is bromocriptine.

6. The use according to claim 1, in which the pharmaceutical composition contains 0.5 to 1.0 milligram of bromocriptine in free base form or in pharmaceutically acceptable acid addition salt form per unit dose.

## Revendications

1. L'utilisation d'un agoniste de la dopamine pour la préparation d'une composition pharmaceutique destinée au traitement de l'accoutumance aux psychostimulants.

2. L'utilisation selon la revendication 1, caractérisée en ce que le psychostimulant est choisi parmi l'amphétamine, la dextroamphétamine, la méthamphétamine et la pémoline ou un sel d'addition d'acide pharmaceutiquement acceptable de ces composés.

3. L'utilisation selon la revendication 1, caractérisée en ce que le psychostimulant est la cocaïne.

4. L'utilisation selon l'une quelconque des revendications 1 à 3, caractérisée en ce que l'agoniste de la dopamine est choisi parmi le lévodopa, le bupropion, le pergolide, le lisuride ou le lergotrile.

5. L'utilisation selon l'une quelconque des revendications 1 à 3, caractérisée en ce que l'agoniste de la dopamine est la bromocriptine.

6. L'utilisation selon la revendication 1, caractérisée en ce que la composition pharmaceutique contient de 0,5 à 1,0 mg de bromocriptine sous forme de base libre ou sous forme d'un sel d'addition d'acide pharmaceutiquement acceptable par dose unitaire.

## Patentansprüche

1. Die Anwendung eines Dopamin-Agonisten für die Herstellung einer pharmazeutischen Zusammensetzung zur Behandlung der durch Psychostimulantien hervorgerufenen Sucht.

2. Die Anwendung gemäss Anspruch 1, worin der Psychostimulant aus Amphetamin, Dextroamphetamin, Methamphetamin und Pemolin oder einem pharmazeutisch verträglichen Säureadditionssalz davon ausgewählt ist.

3. Die Anwendung gemäss Anspruch 1, worin der Psychostimulant Cocain ist.

4. Die Anwendung gemäss irgend einem der Ansprüche 1 bis 3, worin der Dopamin-Agonist aus Levodopa, Bupropion, Pergolid, Lisurid oder Lergotril ausgewählt ist.

5. Die Anwendung gemäss irgend einem der Ansprüche 1 bis 3, worin der Dopamin-Agonist Bromocriptin ist.

6. Die Anwendung gemäss Anspruch 1, worin die pharmazeutische Zusammensetzung 0,5 bis 1,0 Milligramm Bromocriptin als freie Base oder in Form eines pharmazeutisch verträglichen Säureadditionssalzes per Einheitsdosis enthält.

EP 0 204 954 B1

FIGURE I